# EUROPÄISCHE PATENTANMELDUNG

(11) **EP 3 100 690 A1**
(43) Veröffentlichungstag der Anmeldung: **07.12.2016**
(21) Anmeldenummer: 15170269.3
(22) Anmeldetag: 02.06.2015
(51) Int. Cl.: A61B 17/29, A61B 17/00, A61B 90/00

(54) **INSTRUMENT FÜR DIE ENDOSKOPISCHE CHIRURGIE**

(71) Anmelder: RZ-Medizintechnik GmbH, 78532 Tuttlingen (DE)
(72) Erfinder: DOPPELSTEIN, Alexander, 78549 Spaichingen (DE)
(74) Vertreter: Westphal, Mussgnug & Partner Patentanwälte mbB

(57) **Zusammenfassung**

Instrument für die endoskopische Chirurgie, mit einem Schaftrohr (10), mit einer in dem Schaftrohr (10) geführt axial bewegbaren Zugstange (12) und mit distal angeordneten Maulteilen (20), die um einen gemeinsamen Gelenkstift (24) gegeneinander verschwenkbar gelagert sind, wobei die axiale Relativbewegung von Schaftrohr (10) und Zugstange (12) über einen zu dem Gelenkstift (24) exzentrischen Eingriff in die Schwenkbewegung der Maulteile (20) übertragen wird, wobei der Gelenkstift (24) im distalen Ende der Zugstange (12) gelagert ist, wobei die Maulteile (20) jeweils eine zu dem Gelenkstift (24) exzentrische radial vorspringende Nase (34) aufweisen und wobei die Nasen (34) der zwei Maulteile (20) diametral zueinander jeweils in eine Ausnehmung (32) des Schaftrohres (10) eingreifen.

## Beschreibung

Die Erfindung betrifft ein Instrument für die endoskopische Chirurgie gemäß dem Oberbegriff des Patentanspruchs 1.

In der endoskopischen Chirurgie werden Instrumente verwendet, die häufig als Rohrschaftinstrumente bezeichnet werden. Diese Instrumente weisen ein Schaftrohr auf, in welchem axial geführt eine Zugstange bewegbar ist. Ein am proximalen Ende angeordneter Griff, z. B. ein Scherengriff, dient zur axialen Bewegung der Zugstange in dem Schaftrohr, wodurch am distalen Ende angeordnete Maulteile gegeneinander bewegt werden. Die Maulteile können dem unterschiedlichen Anwendungsfalls entsprechend ausgebildet sein, z. B. als Greifer, als Schere, als Koagulationsinstrument und dergleichen.

Bei den herkömmlichen Instrumenten sind üblicherweise die zwei Maulteile auf einem gemeinsamen Gelenkstift schwenkbar gelagert, der in dem distalen Ende des Schaftrohres angeordnet ist. Die Maulteile sind als zweiarmige Hebel ausgebildet, wobei die Zugstange über angelenkte Laschen an den proximalen Schenkelarmen der Maulteile angreift. Eine solche Ausbildung ist z. B. in der DE 93 17 535 U1 gezeigt. Diese Betätigung der Maulteile über doppelte Scherengelenke ist konstruktiv aufwendig. Die Scherengelenke sind für die Reinigung und Sterilisation ungünstig. Außerdem sind die doppelten Scherengelenke nach außen frei zugänglich und können für eine HF-Anwendung nicht isoliert werden.

Eine Verbesserung dieser Probleme wird durch ein Instrument der eingangs genannten Gattung angestrebt, wie es in der DE 10 2010 033 424 A1 beschrieben ist. Bei diesem Instrument sind die zwei Maulteile schwenkbar auf einem gemeinsamen Gelenkstift gelagert, der im distalen Ende des Schaftrohres angeordnet ist. Die in dem Schaftrohr axial bewegbare Zugstange greift an den Maulteilen jeweils exzentrisch zu dem Gelenkstift an, wodurch die Axialbewegung der Zugstange in eine Schwenkbewegung der Maulteile umgesetzt wird. Hierzu wird von dem distalen Ende der Zugstange ein Zugbügel erfasst, der mit abgewinkelten Armen jeweils in eine exzentrische Ausnehmung im Umfang der Maulteile eingreift. Der mehrfach abgewinkelte Zugbügel macht die Herstellung und die Montage des Instruments aufwendig.

Der Erfindung liegt die Aufgabe zugrunde, ein Instrument für die endoskopische Chirurgie der eingangs genannten Gattung im Hinblick auf Herstellung und Montage zu vereinfachen und in Bezug auf die Anwendung zu verbessern.

Diese Aufgabe wird erfindungsgemäß gelöst durch ein Instrument für die endoskopische Chirurgie gemäß dem Patentanspruch 1.

Vorteilhafte Ausführungen und Weiterbildungen der Erfindung sind in den Unteransprüchen angegeben.

Bei dem erfindungsgemäßen Instrument sind die zwei Maulteile um einen gemeinsamen Gelenkstift schwenkbar, der im distalen Ende der Zugstange gelagert ist. Die Umsetzung der axialen Relativbewegung zwischen Zugstange und Schaftrohr in die Schwenkbewegung der Maulteile zum Öffnen und Schließen des Maules wird dadurch bewirkt, dass an den Maulteilen exzentrisch zur Schwenkachse des Gelenkstiftes radial am Umfang des jeweiligen Maulteiles eine Nase absteht, welche in eine Ausnehmung des Schaftrohres eingreift. Die Nase wird in der jeweiligen Ausnehmung des Schaftrohres axial gehalten, so dass die axiale Bewegung des Gelenkstiftes mittels der Zugstange zu einer Dreh- bzw. Schwenkbewegung des jeweiligen Maulteiles führt.

Das Instrument ist im Hinblick auf Herstellung und Montage einfach. Es müssen lediglich die Maulteile mittels des Gelenkstiftes im distalen Ende der Zugstange angeordnet werden, ohne dass zusätzliche Bauteile für das distale Arbeitsende des Instruments erforderlich sind. Die Zugstange mit den Maulteilen wird in das Schaftrohr eingesetzt, so dass die Nasen der Maulteile in die zugehörigen Ausnehmungen des Schaftrohres eingreifen. Aus dem distalen Ende des Schaftrohres ragen lediglich die Arbeitsenden der Maulteile heraus, während sich der Betätigungs- und Schwenkmechanismus im distalen Ende des Schaftrohres befindet. Die Maulteile können daher gut gereinigt und sterilisiert werden. Das distale Ende des Schaftrohres umgreift das Schwenkgelenk der Maulteile und deren Betätigung, so dass eine elektrische Isolierung am Außenumfang des Schaftrohres distal bis an die freiliegenden Arbeitsenden der Maulteile geführt werden kann. Ist das Instrument für eine HF-Funktion ausgebildet, z. B. als Koagulationsinstrument, so ist die freiliegende Elektrodenfläche der Maulteile vorteilhaft klein für eine gezielte definierte Hochfrequenz-Einwirkung auf das Gewebe.

Die Ausnehmungen, in welche die Nasen der Maulteile eingreifen, können als Durchbrüche in der Wandung des Schaftrohres ausgebildet sein. In einer vorteilhaften Ausführung sind dagegen diese Ausnehmungen in der Wandung einer Hülse ausgebildet, die koaxial in das distale Ende des Schaftrohres eingesetzt und in diesem befestigt wird. Die als Durchbrüche der Wandung der Hülse ausgebildeten Ausnehmungen werden dabei am Außenumfang durch das Schaftrohr abgedeckt und verschlossen. Das bis zum distalen Ende am Außenumfang vollständig geschlossene Schaftrohr gewährleistet dabei, dass keine Verschmutzungen in den Schwenkmechanismus eindringen können. Außerdem kann eine eventuelle elektrische Isolierung am Außenumfang des Schaftrohres vollständig und ohne Unterbrechung bis an das distale Ende des Schaftrohres geführt werden.

In einer weiteren vorteilhaften Ausführung kann der Gelenkstift, auf welchem die Maulteile schwenkbar gelagert sind, mit einem Ende oder mit beiden Enden über den Außenumfang der Zugstange hinausragen. Mit diesem hinausragenden Ende greift der Gelenkstift in einen axialen Schlitz ein, der in dem Schaftrohr oder bei Verwendung einer Hülse vorzugsweise in der Hülse ausgebildet ist. Der in den axialen Schlitz eingreifende Gelenkstift verhindert ein Verdrehen der Zugstange und damit der in der Zugstange gelagerten Maulteile gegenüber dem Schaftrohr. Die Zuordnung der Schwenkebene der Maulteile in Bezug auf den proximalen Griff des Instruments ist dadurch eindeutig festgelegt, was für die Benutzung des Instruments von größter Bedeutung ist.

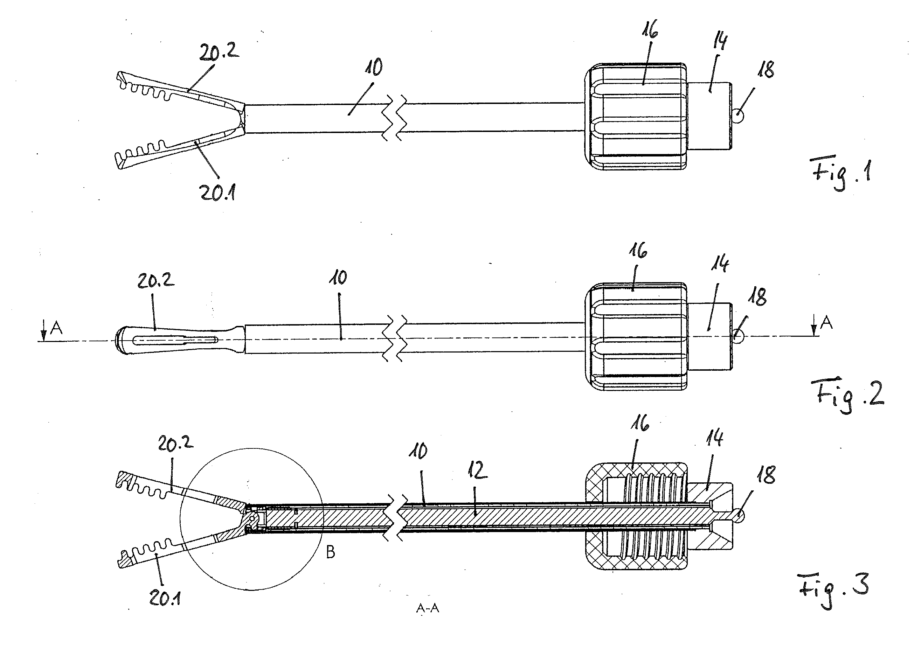

Im Folgenden wird die Erfindung anhand eines in der Zeichnung dargestellten Ausführungsbeispiels näher erläutert, woraus weitere erfindungswesentliche Merkmale und Vorteile deutlich werden. Dabei zeigen
- Figur 1: eine Seitenansicht des Instruments,
- Figur 2: eine um 90° gedrehte Seitenansicht des Instruments,
- Figur 3: einen Axialschnitt durch das Instrument gemäß der Schnittlinie A - A in Figur 2,
- Figur 4: eine vergrößerte Detail-Darstellung gemäß dem Ausschnitt B in Figur 3,

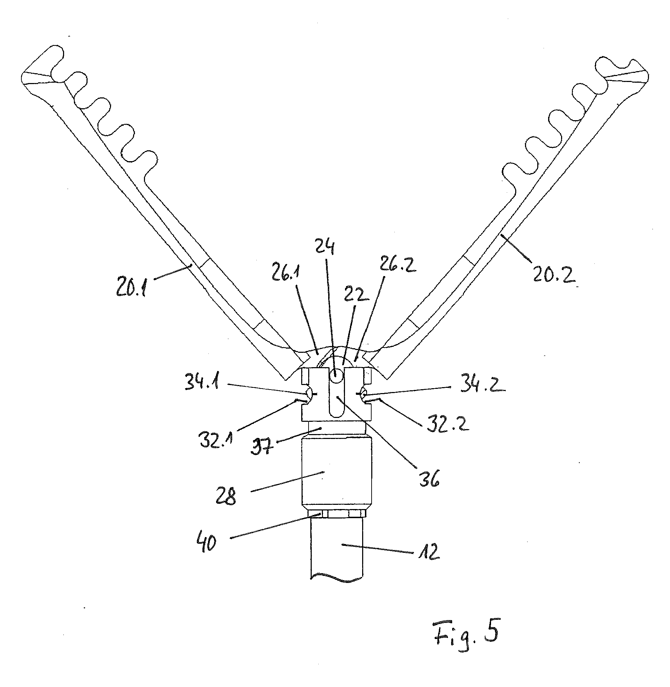

- Figur 5: eine Seitenansicht des distalen Endes des Instruments, wobei das Schaftrohr entfernt ist,
- Figur 6: eine perspektivische Ansicht entsprechend Figur 5 und
- Figur 7: eine perspektivische Einzeldarstellung der Hülse.

Das erfindungsgemäße Instrument für die endoskopische Chirurgie weist ein starres Schaftrohr 10 auf, in welchem axial beweglich eine Zugstange 12 geführt ist. Am proximalen Ende des Instrumentes ist in an sich bekannter Weise ein Griff angeordnet, der in der Zeichnung nicht dargestellt ist. Der Griff, der beispielsweise als Scherengriff ausgebildet sein kann, weist ein starres Griffteil und ein zur Betätigung des Instruments bewegbares Griffteil auf. Das Schaftrohr 10 wird mit seinem proximalen Ende mittels einer Anschlagbuchse 14 und einer Überwurfmutter 16 mit dem starren Griffteil verbunden. Die Zugstange 12 weist an ihrem proximalen Ende einen Kugelkopf 18 auf, der in dem bewegbaren Griffteil aufgenommen wird, so dass durch Betätigung des bewegbaren Griffteils die Zugstange 12 axial in dem Schaftrohr 10 verschoben werden kann.

Am distalen Ende weist das Instrument zwei Maulteile 20.1 und 20.2 auf, die durch die axiale Verschiebung der Zugstange 12 in einer Schließbewegung gegeneinander geschwenkt bzw. zum Öffnen auseinander geschwenkt werden können. Die Schließbewegung ist dabei die aktive Betätigung des Instruments, bei welcher eine größere Kraftwirkung durch die Maulteile 20 erforderlich ist. Die Schließbewegung wird daher vorzugsweise durch eine proximalwärts gerichtete Zugkraft der Zugstange 12 bewirkt, weshalb diese als "Zugstange" bezeichnet wird. Die distalwärts gerichtete Bewegung der Zugstange 12 erzeugt eine distalwärts gerichtete Schubkraft, die zum Aufspreizen der Maulteile 20 führt. Die Maulteile 20 können je nach Verwendungszweck des Instruments in an sich bekannter Weise ausgebildet sein. Im dargestellten Ausführungsbeispiel sind die Maulteile 20 als Greifzange ausgebildet. Ebenso können die Maulteile 20 als Schere, als Klemme oder als Koagulationsklemme ausgebildet sein.

Das distale Ende der Zugstange 12 ist als distalwärts offene Gabel 22 ausgebildet. Die Gabel 22 ist von einer senkrecht zur Achse der Zugstange 12 verlaufenden Bohrung durchsetzt, in welche ein Gelenkstift 24 eingesetzt wird. Die Maulteile 20.1 und 20.2 sind an ihrem proximalen Ende als flache Scheibe 26.1 bzw. 26.2 ausgebildet. Die Scheiben 26.1 und 26.2 sind nebeneinander und mit den Flachseiten aneinander anliegend in die Gabel 22 eingesetzt. Der Gelenkstift 24 durchsetzt die Scheiben 26.1 und 26.2 zentrisch, so dass die Maulteile 20.1 und 20.2 in der Ebene der Scheiben 26 schwenkbar auf dem Gelenkstift 24 gelagert sind.

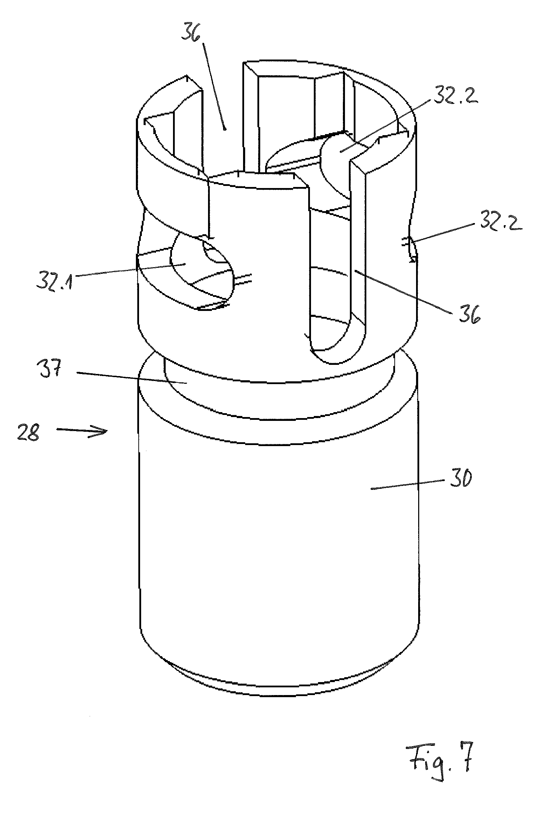

Auf dem distalen Ende der Zugstange 12 sitzt axial frei verschiebbar eine Hülse 28, die in Figur 7 als Einzelteil dargestellt ist. Die Hülse 28 ist koaxial in das distale Ende des Schaftrohres 10 eingesetzt und in diesem Schaftrohr 10 axial und gegen Drehung fixiert. Hierzu ist die Hülse 28 in ihrem proximalen Endabschnitt 30 mit einem Außengewinde 31 versehen, welches in ein korrespondierendes Innengewinde des Schaftrohres 10 eingeschraubt wird. Andere Fixierungen der Hülse 28 in dem Schaftrohr 10 sind ebenfalls möglich. Der Endabschnitt 30 der Hülse 28 kann beispielsweise mittels eines Bajonettverschlusses, durch Verkleben, durch Einpressen oder in sonstiger Weise in dem Schaftrohr 10 fixiert werden.

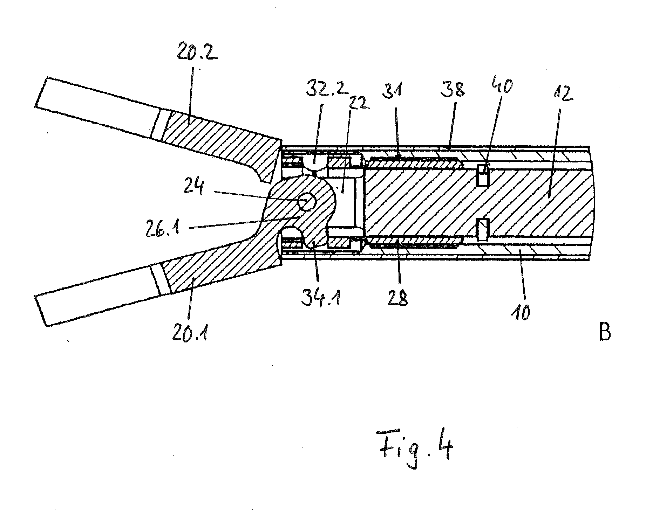

Die Hülse 28 weist in ihrem distalen Endbereich zwei Ausnehmungen 32.1 und 32.2 auf, die als diametral angeordnete Wanddurchbrüche der Hülse 28 ausgebildet sind. An den Scheiben 26.1 und 26.2 der Maulteile 20.1 und 20.2 ist jeweils eine Nase 34.1 bzw. 34.2 angeformt. Die Nasen 34.1 und 34.2 sind jeweils am Außenumfang der Scheiben 26.1 bzw. 26.2 exzentrisch zu dem Gelenkstift 24 angeformt und ragen im Wesentlichen radial vom Umfang der Scheiben 26.1 bzw. 26.2 ab. Die Nasen 34.1 bzw. 34.2 sind dabei so am Umfang der Scheiben 26.1 bzw. 26.2 angeordnet, dass sie diamtral zueinander vom Gelenkstift 24 nach außen gerichtet sind. Die Maulteile 20.1 und 20.2 greifen mit ihren jeweiligen Nasen 34.1 bzw. 34.2 in die Ausnehmungen 32.1 bzw. 23.2 der Hülse 28, wie dies am Deutlichsten in Figur 4 zu sehen ist.

Wird bei der Benutzung des Instruments die Zugstange 12 mittels des nicht dargestellten Griffs axial in dem Schaftrohr 10 distalwärts vorgeschoben, so bewegt sich der Gelenkstift 24, auf welchem die Maulteile 20.1 und 20.2 gelagert sind distalwärts in Bezug auf das Schaftrohr 10 und die fest in dem Schaftrohr 10 sitzende Hülse 28. Die Nasen 34.1 und 34.2 der Maulteile 20.1 bzw. 20.2 werden dabei in den jeweiligen Ausnehmungen 32.1 bzw. 32.2 der Hülse 28 axial festgehalten. Durch die exzentrische Anordnung der Nasen 34.1 und 34.2 werden dadurch die Maulteile 20.1 und 20.2 um den Gelenkstift 24 geschwenkt. Wie in Figur 4 zu sehen ist, wird dabei das Maulteil 20.1 durch die in die Ausnehmung 32.1 eingreifende Nase 34.1 entgegen dem Uhrzeigersinn geschwenkt, während das Maulteil 20.2 durch die in die Ausnehmung 32.2 eingreifende Nase 34.2 im Uhrzeigersinn geschwenkt wird. Die distalwärts gerichtete Vorschubbewegung der Zugstange 12 führt somit zu einem Auseinanderspreizen der Maulteile 20.1 und 20.2. Wird umgekehrt die Zugstange 12 mittels des nicht dargestellten Griffes in dem Schaftrohr 10 proximalwärts gezogen, so bewegt sich der Gelenkstift 24 mit den Maulteilen 20.1 und 20.2 in die Hülse 28 hinein. Die in die axial feststehenden Ausnehmungen 32.1 und 32.2 eingreifenden Nasen 34.1 und 34.2 werden im entgegengesetzten Sinn um den Gelenkstift 24 geschwenkt, so dass das Maulteil 20.1 in der Darstellung der Figur 4 im Uhrzeigersinn und das Maulteil 20.2 im Gegenuhrzeigersinn geschwenkt, so dass das durch diese Maulteile gebildete Maul geschlossen wird.

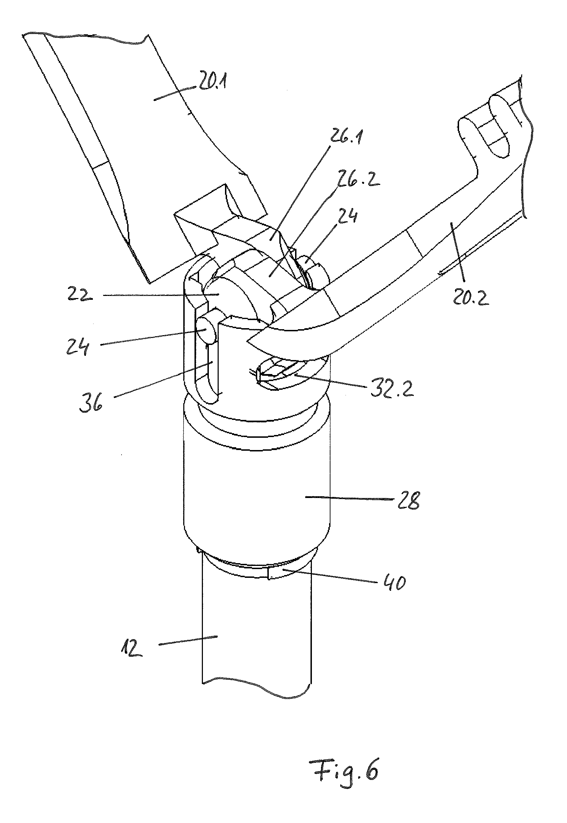

Die Hülse 28 weist an ihrem distalen Ende wenigstens einen, im dargestellten Ausführungsbeispiel zwei diametral angeordnet axiale Schlitze 36 auf, die gegen den distalen Rand der Hülse 28 offen sind. Die Schlitze 36 sind gegen die Ausnehmungen 32 jeweils um 90° versetzt. Wie am Deutlichsten in Figur 6 zu erkennen ist, ist der Gelenkstift 24 an einem Ende, vorzugsweise an seinen beiden Enden über den Außenumfang der Zugstange 12 hinaus verlängert. Mit diesem verlängerten Ende greift der Gelenkstift 24 radial in die Schlitze 36 ein. Die axiale Länge der Schlitze 36 ermöglicht die axiale Bewegung der Zugstange 12 mit dem Gelenkstift 24 in der fest in dem Schaftrohr 10 sitzenden Hülse 28. Der Eingriff des Gelenkstiftes 24 in die Schlitze 36 bewirkt dabei, dass die Zugstange 12 in dem Schaftrohr 10 unverdrehbar geführt ist. Dadurch ist die Schwenkebene der in der Zugstange 12 gelagerten Maulteile 20 unverdrehbar in Bezug auf das Schaftrohr 10 und den mit dem Schaftrohr verbundenen Griff festgelegt.

Die Montage des Instruments wird in folgender Weise durchgeführt:
Zunächst werden die Maulteile 20.1 und 20.2 in die Gabel 22 der Zugstange 12 eingesetzt und mittels des Gelenkstiftes 24 in der Zugstange gelagert. Anschließend wird die Hülse 28 auf der Zugstange 12 distalwärts gegen die Maulteile 20 vorgeschoben. Dabei werden die Maulteile 20 soweit auseinander gespreizt, dass die Nasen 34.1 und 34.2 vollständig in den freien Innenquerschnitt der Hülse 28 eintreten. Die Hülse 28 kann somit über die nach innen geschwenkten Nasen 34 geschoben werden. Sobald die Hülse 28 mit den Ausnehmungen 32.1 und 32.2 in der axialen Position der Nasen 34.1 und 34.2 ist, werden die Maulteile 20.1 und 20.2 soweit zusammen geschwenkt, dass die Nasen 34.1. und 34.2 in die jeweiligen Ausnehmungen 32.1 und 32.2 der Hülse 28 eingreifen. Anschließend wird die Zugstange 12 mit der Hülse 28 in das Schaftrohr 10 eigeführt. Die Hülse 28 wird dabei durch die in die Ausnehmungen 32 eingreifenden Nasen 34 axial auf der Zugstange 12 gehalten, während der in die Schlitze 36 eingreifende Gelenkstift 24 die Hülse verdrehfest auf der Zugstange 12 hält. Mittels der Zugstange 12 kann somit die Hülse 28 mit dem Gewinde 31 ihres Endabschnittes 30 in das Innengewinde des Schaftrohres 10 eingedreht werden, um die Hülse 28 in dem Schaftrohr 10 zu fixieren. Ein das distale Ende des Gewindes des Endabschnittes 30 begrenzender Einstich 37 dient dabei als Anschlag, der die axiale Position der Hülse 28 in dem Schaftrohr 10 definiert.

Wenn die Hülse 28 vollständig in das distale Ende des Schaftrohres 10 eingesetzt ist, greift das Schaftrohr 10 über die Wanddurchbrüche der Ausnehmungen 32 und der Schlitze 36, so dass diese am Außenumfang durch das Schaftrohr 10 vollständig abgedeckt und verschlossen sind. Das Schaftrohr 10 ist somit mit vollständig geschlossener Wandung distal bis an die Maulteile 20 geführt. Vorzugsweise kann die äußere Mantelfläche des Schaftrohres 10 durch eine elektrische Isolierung 38 isoliert sein, die ebenfalls durchgehend distal bis an die Maulteile 20 geführt ist. Hierdurch eignet sich das Instrument insbesondere für Hochfrequenz-Anwendungen.

Bevor die Zugstange 12 mit der aufgeschobenen Hülse 28 in das Schaftrohr 10 eingesetzt wird, wird vorzugsweise proximal hinter der Hülse 28 eine Wellensicherung 40 in einen Einstich der Zugstange 12 eingesetzt. Die Wellensicherung 40 ist axial frei bewegbar in dem Schaftrohr 10, bildet jedoch bei der distalwärts gerichteten Vorschubbewegung der Zugstange 12 einen Anschlag an der in dem Schaftrohr 10 festsitzenden Hülse 28. Die Wellensicherung 40 begrenzt auf diese Weise die distal gerichtete Vorschubbewegung der Zugstange 12. Dadurch wird verhindert, dass die Zugstange 12 distalwärts soweit vorgeschoben werden kann, bis die Maulteile 20.1 und 20.2 maximal auseinander gespreizt sind und die Nasen 34.1 und 34.2 nach innen aus den Ausnehmungen 32.1 und 32.2 austreten, wodurch die Zugstange 12 mit den Maulteilen 20 nicht mehr distalwärts in dem Schaftrohr 10 gehalten wäre.

### Bezugszeichenliste

- 10: Schaftrohr
- 12: Zugstange
- 14: Anschlagbuchse
- 16: Überwurfmutter
- 18: Kugelkopf
- 20: Maulteile
- 22: Gabel
- 24: Gelenkstift
- 26: Scheiben
- 28: Hülse
- 30: Endabschnitt
- 31: Gewinde
- 32: Ausnehmungen
- 34: Nasen
- 36: Schlitze
- 37: Einstich
- 38: Isolierung
- 40: Wellensicherung

## Patentansprüche

1. Instrument für die endoskopische Chirurgie, mit einem Schaftrohr (10), mit einer in dem Schaftrohr (10) geführt axial bewegbaren Zugstange (12) und mit distal angeordneten Maulteilen (20), die um einen gemeinsamen Gelenkstift (24) gegeneinander verschwenkbar gelagert sind, wobei die axiale Relativbewegung von Schaftrohr (10) und Zugstange (12) über einen zu dem Gelenkstift (24) exzentrischen Eingriff in die Schwenkbewegung der Maulteile (20) übertragen wird,
**dadurch gekennzeichnet, dass** der Gelenkstift (24) im distalen Ende der Zugstange (12) gelagert ist, dass die Maulteile (20) jeweils eine zu dem Gelenkstift (24) exzentrische radial vorspringende Nase (34) aufweisen und dass die Nasen (34) der zwei Maulteile (20) diametral zueinander jeweils in eine Ausnehmung (32) des Schaftrohres (10) eingreifen.

2. Instrument nach Anspruch 1,
**dadurch gekennzeichnet, dass** die Ausnehmungen (32) in einer Hülse (28) ausgebildet sind, die koaxial fest in das distale Ende des Schaftrohres (10) eingesetzt ist.

3. Instrument nach Anspruch 2,
**dadurch gekennzeichnet, dass** die Ausnehmung (32) der Hülse (28) am Außenumfang durch das Schaftrohr (10) abgedeckt und geschlossen sind.

4. Instrument nach Anspruch 2,
**dadurch gekennzeichnet, dass** auf der Zugstange (12) eine Wellensicherung (40) angeordnet ist, die zur Begrenzung der distalwärts gerichteten Vorschubbewegung der Zugstange (12) an der Hülse (28) zum Anschlag kommt.

5. Instrument nach Anspruch 2,
**dadurch gekennzeichnet, dass** die Hülse (28) in das Schaftrohr (10) eingeschraubt, eingepresst oder eingeklebt ist.

6. Instrument nach Anspruch 5,
**dadurch gekennzeichnet, dass** die Hülse (28) mit einem Außengewinde (31) in ein Innengewinde des Schaftrohres (10) eingeschraubt ist, wobei das Gewinde die definierte axiale Position der Hülse (28) in dem Schaftrohr (10) festlegt.

7. Instrument nach einem der vorhergehenden Ansprüche,
**dadurch gekennzeichnet, dass** der Gelenkstift (24) mit wenigstens einem Ende über den Umfang der Zugstange (12) hinausragt und mit diesem Ende in einen axialen Schlitz (36) des Schaftrohres (10) bzw. der Hülse (28) eingreift, wodurch die Zugstange (12) mit den Maulteilen (20) unverdrehbar in dem Schaftrohr (10) geführt ist.

8. Instrument nach Anspruch 7,
**dadurch gekennzeichnet, dass** der wenigstens eine axiale Schlitz (36) in der Hülse (28) ausgebildet ist und am Außenumfang durch das Schaftrohr (10) abgedeckt ist.

9. Instrument nach einem der vorhergehenden Ansprüche, **dadurch gekennzeichnet, dass** das Schaftrohr (10) an seinem Außenumfang bis an sein an die Maulteile (20) angrenzendes distales Ende elektrisch isoliert ist.
